(19) Europäisches Patentamt
European Patent Office
Office européen des brevets

(11) Veröffentlichungsnummer: **0 043 346**
**A1**

(12) # EUROPÄISCHE PATENTANMELDUNG

(21) Anmeldenummer: **81810257.6**

(22) Anmeldetag: **22.06.81**

(51) Int. Cl.$^3$: **C 08 G 59/30**
C 08 K 5/34, C 07 D 235/26

(30) Priorität: **26.06.80 CH 4914/80**

(43) Veröffentlichungstag der Anmeldung:
**06.01.82 Patentblatt 82/1**

(84) Benannte Vertragsstaaten:
**CH DE FR GB LI**

(71) Anmelder: **CIBA-GEIGY AG**
**Patentabteilung Postfach**
**CH-4002 Basel(CH)**

(72) Erfinder: **Buxbaum, Lothar, Dr.**
**Moosschmiedenweg**
**A-9500 Villach/Sankt Georgen(AT)**

(54) **Oligomere Epoxidharze und deren Verwendung als Flammschutzmittel.**

(57) Oligomere, im wesentlichen lineare Epoxidharze mit chlor- und/oder bromhaltigen Benzimidazolonresten in der Oligomerkette eignen sich gut als Flammschutzmittel für duroplastische und thermoplastische Polymere.

EP 0 043 346 A1

Croydon Printing Company Ltd.

CIBA-GEIGY AG
Basel (Schweiz)

3-12929/=

# Oligomere Epoxidharze und deren Verwendung als Flammschutzmittel

Die vorliegende Erfindung betrifft halogenhaltige, im wesentlichen lineare, oligomere Epoxidharze, die halogenierte Benzimidazolongruppen enthalten, und deren Verwendung als Flammschutzmittel in Polymeren.

Es sind bereits viele halogenhaltige organische Verbindungen als Flammschutzmittel für Polymere vorgeschlagen worden, die entweder allein oder üblicherweise zusammen mit Synergisten wie N-, P- oder Antimonverbindungen eingesetzt werden. Die bekannten halogenhaltigen Verbindungen können nicht immer den Anforderungen genügen, die an sie hinsichtlich des Extraktions- und Migrationsverhaltens, der thermischen Beständigkeit, des negativen Einflusses auf die Substrateigenschaften und des Korrosionseinflusses des flammwidrig ausgerüsteten Substrates auf Kontaktmetalle gestellt werden.

Aufgabe vorliegender Erfindung ist es, Flammschutzmittel bereitzustellen, die diesen Anforderungen genügen.

Gegenstand der vorliegenden Erfindung sind halogenhaltige, im wesentlichen lineare, oligomere Epoxidharze der Formel I

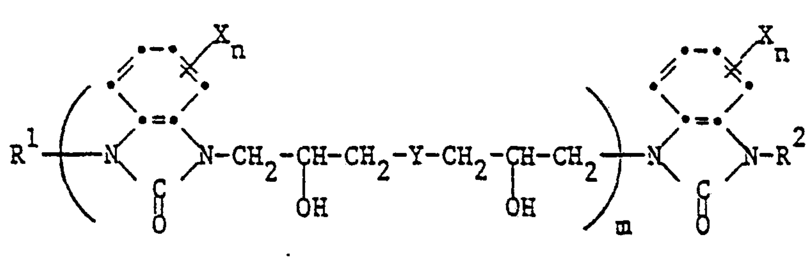

(I)

worin n die Zahlen 2, 3 oder 4 und m ein Zahlenwert von 1 bis 30 sind, X für Chlor oder Brom steht, Y den zweiwertigen Rest einer H-aciden Verbindung aus der Gruppe der Diole, zweiwertigen Phenole, Bisphenole, Dicarbonsäuren oder cyclischen Ureide darstellt und $R^1$ und $R^2$ für ein H-Atom, $-CH_2-\underset{O}{CH-CH_2}$ oder $-CH_2-\underset{OH}{CH}-CH_2-Y-H$ stehen.

Bevorzugt ist in Formel I n = 3 und besonders 4 und m ein Zahlenwert von 1 bis 20, besonders 1 bis 10. X steht vorzugsweise für Brom.

Y in Formel I kann der zweiwertige Rest eines Dioles mit bevorzugt 2-12 C-Atomen sein. Es kann sich um ein lineares, verzweigtes und durch N-Heterocyclische Gruppe, Cycloalkylengruppen oder O-Atome unterbrochenes Alkylendiol oder um ein cycloaliphatisches Diol handeln. Beispiele sind Aethylenglykol, 1,2- oder 1,3-Propandiol, 1,2-, 1,3- oder 1,4-Butandiol, 1,5-Pentandiol, Hexandiol, Decandiol, Dodecandiol, Diäthylenglycol, Triäthylenglykol, Diopropylenglykol, 1,4-Dihydroxymethylcyclohexan, 1,4-Dihydroxycyclohexan, N,N-(β-hydroxyäthyl)-5,5-dimethylhydantoin, N,N-(β-Hydroxyäthyl)-tetrachlor- oder tetrabrombenzimidazolon.

Y in seiner Bedeutung als zweiwertiger Rest einer Dicarbonsäure kann sich von aliphatischen cycloaliphatischen und aromatischen Dicarbonsäuren ableiten, die bevorzugt 2 bis 20 und insbesondere 4 bis 16 C-Atome aufweisen. Beispiele sind: Oxalsäure, Malonsäure, Aethylmalonsäure, Dodecylmalonsäure, Bernsteinsäure, Decylbernsteinsäure, Glutarsäure, Adipinsäure, Trimethyladipinsäure, Pimelinsäure, Suberinsäure, Azelainsäure, Sebacinsäure, Octadecandicarbonsäure, Dimersäuren (Dimerisationsprodukte olefinisch ungesättigter $C_{10}$-$C_{20}$-Fettsäuren), Cyclohexan-1,4-dicarbonsäure, Phthalsäure, Isophtalsäure, Terephthalsäure, 4,4'-Diphenyldicarbonsäure, 4,4'-Carboxyldiphenyläther.

Y in seiner Bedeutung als Rest eines zweiwertigen Phenoles kann unsubstituiertes oder $C_1$-$C_4$-alkylsubstituiertes Hydrochinon, Brenzcatechin oder Resorcin sein.

Y als zweiwertiger Rest eines Bisphenoles entspricht bevorzugt der Formel II

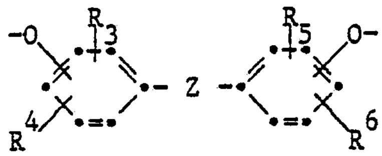 (II)

worin $R^3$, $R^4$, $R^5$ und $R^6$ unabhängig voneinander ein Wasserstoffatom, Alkyl mit 1-4 C-Atomen, ein Chlor- oder ein Bromatom darstellt und Z für eine direkte Bindung, O, S, SO, $SO_2$, $CO_2$, Alkylen mit 1 bis 8 C-Atomen, Alkyliden mit 2-8 C-Atomen oder Cylcoalkyliden mit 5 oder 6 Ring-C-Atomen steht.

Die Substituenten $R^3$ bis $R^6$ sind bevorzugt in Orthostellung zum Sauerstoffatom gebunden und das Sauerstoffatom bevorzugt in Meta- und besonders Parastellung zur Gruppe Z. Die Gruppe Z enthält in der Bedeutung als Alkylen bevorzugt 1 bis 4 C-Atome und in der Bedeutung als Alkyliden bevorzugt 2-6 C-Atome. Beispiele für Alkylen sind Methylen, Aethylen, 1,2- oder 1,3-Propylen, Butylen und Hexylen. Beispiele für Alkyliden und Cycloalkyliden sind Aethyliden, 2-Chlor-1,1-äthyliden, 2,2-Dichlor-1,1-äthyliden, 2,2,2-Trichlor-1,1-äthyliden, 1,1- oder 2,2-Propyliden, 1,1- oder 2,2-Butyliden, Pentyliden, Hexyliden, Octyliden, Cyclopentyliden, Cyclohexyliden.

Beispiele sind 4,4'-Dihydroxydiphenyl, Bis(p-hydroxyphenyl)-methan, 1,1-Bis(p-hydroxyphenyl)cyclohexan, 2,2-Bis(p-hydroxyphenyl) propan, 2,2-Bis(4-hydroxy-3,5-dibromphenyl)propan, 4,4'- Dihydroxydiphenyläther. Weitere Beispiele sind in der DE-OS 26 01 960 aufgezählt.

Y als zweiwertiger Rest eines cyclischen Ureides entspricht bevorzugt den Formeln III - VII,

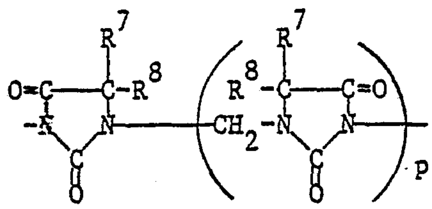 (III), 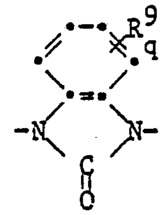 (IV)

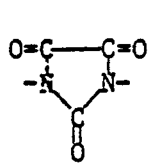 (V), 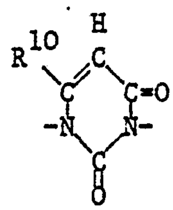 (VI), 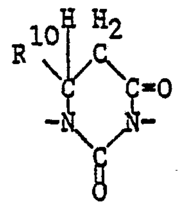 (VII),

worin p 0 oder 1 und q 0 oder ganze Zahlen von 1-4 bedeuten, $R^7$ oder $R^8$ ein Wasserstoffatom, Phenyl oder $C_1$-$C_{12}$-Alkyl oder $R^7$ und $R^8$ zusammen Tetra- oder Pentamethylen sind, $R^9$ Methyl, Chlor oder Brom bedeutet und $R^{10}$ für ein Wasserstoffatom oder $C_1$-$C_4$ Alkyl steht.

$R^7$ oder $R^8$ in Formel II sind bevorzugt ein Wasserstoffatom, Phenyl oder $C_1$-$C_4$-Alkyl wie Methyl, Aethyl, n-Propyl, i-Propyl, n-Butyl, i-Butyl oder t-Butyl. In Formel IV ist q bevorzugt 3 und besonders 4 und $R^9$ steht bevorzugt für Chlor und besonders Brom. Beispiele für $R^{10}$ sind Butyl, n-Propyl, Aethyl, Methyl, bevorzugt ist $R^{10}$ ein Wasserstoffatom oder Methyl.

Als besonders wertvoll haben sich Epoxidharze der Formel I erwiesen, worin n = 4 und m ein Zahlenwert von 1-10 sind, X für Brom steht und Y einen Rest der Formel IV darstellt, worin $R^9$ für Chlor und besonders Brom steht und q die Zahlen 3 und besonders 4 bedeutet.

Die Herstellung der erfindungsgemässen Epoxidharze erfolgt nach bekannten Verfahren des Standes der Technik in hierfür üblichen technischen Einrichtungen.

In einer Ausführungsform kann ein chloriertes oder bromiertes N,N'-Diglycidylbenzimidazolon der Formel

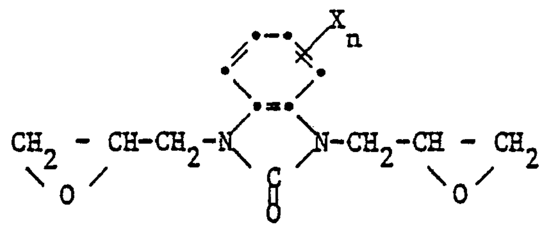

mit einer bifunktionellen H-aciden Verbindung H-Y-H umgesetzt werden, wobei X, n und y die zuvor angegebene Bedeutung haben.

Diese Umsetzung, die als Advancement-Verfahren im US-Patent 3 306 872 beschrieben ist, kann ohne oder in Gegenwart eines vorzugsweise polaren aprotischen Lösungsmittels wie Aethern (Tetrahydrofuran, Dioxan, Dibutyläthern) Sulfonen (Dimethylsulfoxid), Ketonen oder Carbonsäureamiden (Dimethylformamid) durchgeführt werden. Das molare Verhältnis von Diglycidylverbindung zu H-acider Verbindung kann hierbei von 2:1 bis 1:2, vorzugsweise 1,5:1 bis 1:1,5 sein. Die Reaktionstemperatur beträgt von 100 bis 250°C und die Reaktionszeit kann bis 5 Stunden und mehr betragen, je nach gewünschtem Oligomerisationsgrad, der über die Viskosität des Reaktionsproduktes bestimmt werden kann.

Die Umsetzung kann mit Katalysatoren beschleunigt werden. Solche Härtungsbeschleuniger werden im allgemeinen in Mengen von 0,1 bis 5, vorzugsweise 0,1 bis 2 Gew. % eingesetzt, bezogen auf die Reaktanden. Geeignet sind jene Verbindungen, die die Härtungsreaktion zu beschleunigen vermögen. Beispiele sind:

Imidazol und seine Homologen sowie deren Salze mit Polycarbonsäuren bzw. deren Anhydriden wie Imidazol, 1-Methylimidazol, 2-Aethylimidazol, 2-Methyl-4-äthylimidazol, 2-Phenylimidazol, Benztriazol, organische Phosphorverbindungen, $BF_3$- oder $BCl_3$-Komplexe, organische Phosphoniumborate, Alkylammoniumhalogenide (z.B. die Chloride und Bromide) wie Tetramethylammoniumchlorid, Harnstoffe und Derivate, wie N-p-Chlorphenyl-N,N'-dimethylharnstoff. Bevorzugt sind die Imidazole.

In einer weiteren Ausführungsform zur Herstellung der erfindungsgemässen oligomeren können unter ähnlichen Reaktionsbedingungen auch die Bisglycidylderivate der H-aciden Verbindungen mit der Formel

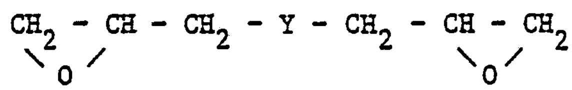

mit chlorierten oder bromierten Benzimidazolen der Formel

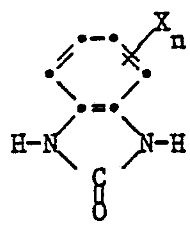

umgesetzt werden. Auch bei dieser Umsetzung handelt es sich um ein Advancementverfahren, wie im US Patent 3 306 872 beschrieben.

In einer anderen Ausführungsform zur Herstellung der erfindungsgemässen Oligomeren kann man auch ein chloriertes oder bromiertes Benzimidazolon und eine H-acide Verbindung direkt mit Epichlorhydrin umsetzen (Taffy-Verfahren, vergleiche U.S. Patent 2 602 075).

Die als Ausgangsprodukte verwendeten partiell chlorierten Benzimidazolonbisglycide sind aus der DE-OS 2 300 010 bekannt. Die partiell bromierten sowie die vollständig chlorierten oder bromierten Benzimidazolonbisglycide sind neu und werden nach analogen Verfahren hergestellt.

Bei den erfindungsgemässen Epoxidharzen handelt es sich um im wesentlichen lineare Oligomere. Vernetzungen über die bei der Additionsreaktion gebildete sekundäre OH-Gruppe werden nur im ganzen Umfang beobachtet. Sie habeneine flüssige bis viskose oder harzartige Konsistenz, die eine relative Viskosität bis 1,3 aufweisen können. Die hochschmelzenden Verbindungen haben auch eine ausgezeichnete thermische Stabilität.

Die erfindungsgemässen Epoxidharze eignen sich hervorragend als Flammschutzmittel für Kunststoffe, da sie wenig korrosive Dämpfe bei Erwärmung entwickeln und daher Kontaktmetalle weniger schädigen. Auch ihre hohe Migrationsbeständigkeit stellt einen erheblichen Vorteil dar. Ferner ist die in der Schwerlöslichkeit der Verbindungen begründete Extraktionsbeständigkeit hervorzuheben.

Ein weiterer Gegenstand vorliegender Erfindung ist eine flammwidrige Kunststoffmasse, die einen duroplastischen, oder thermoplastischen Kunststoff und eine wirksame Menge eines Epoxidharzes der Formel I als Flammschutzmittel enthält.

Im allgemeinen werden dem Kunststoff Mengen von 0,1 bis 30 Gew.-% bevorzugt 1 bis 15 Gew.-% einverleibt, bezogen auf den Kunststoff. Das Flammschutzmittel kann alleine oder bevorzugt zusammen mit etwa 0,1 bis 20, besonders 1 bis 15 Gew.-%, bezogen auf den Kunststoff, einer synergistisch wirkenden Verbindung eines Elementes der fünften Hauptgruppe des Periodensystems verwendet werden. Neben Stickstoff- oder Phosphorverbindungen sind besonders Antimonverbindungen wie z.B. Antimontrioxid geeignet.

Als Kunststoffe kommen sowohl Duroplaste und Thermoplaste in Frage. Die thermoplastischen Kunststoffe sind bevorzugt.

Beispiele sind:

1. Polymere, die sich von einfach oder doppelt ungesättigten Kohlenwasserstoffen ableiten, wie Polyolefine, wie z.B. Polyäthylen, das gegebenenfalls vernetzt sein kann, Polypropylen, Polyisobutylen, Polymethylbuten-1, Polymethylpenten-1, Polybuten-1, Polyisopren, Polybutadien, Polystyrol, Polyisobutylen, Copolymere der den genannten Homopolymeren zugrundeliegenden Monomeren, wie Aethylen-Propylen-Copolymere, Propylen-Buten-1-Copolymere, Propylen-Isobutylen-Copolymere, sowie Terpolymere von Aethylen und Propylen mit einem Dien, wie z.B. Hexadien, Dicyclopentandien oder Aethylidennerbornen;

Mischungen der oben genannten Homopolymeren, wie beispielsweise Gemische von Polypropylen und Polyäthylen, Polypropylen und Poly-Buten-1, Polypropylen und Polyisobutylen.

2. Halogenhaltige Vinylpolymere, wie Polyvinylchlorid, Polyvinylidenchlorid, Polyvinylfluorid, aber auch Polychloropren und Chlorkautschuke.

3. Polymere, die sich von α,β-ungesättigten Säuren und deren Derivaten ableiten, wie Polyacrylate und Polymethacrylate, Polyacrylamide und Polyacrylnitril, sowie deren Copolymere mit anderen Vinylverbindungen, wie Acrylnitril/Butadien/Styrol, Acrylnitril/Styrol und Acrylnitril/Styrol/Acrylester-Copolymerisate.

4. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin und deren Copolymere mit anderen Vinylverbindungen, wie Aethylen/Vinylacetat-Copolymere.

5. Homo- und Copolymere, die sich von Epoxyden ableiten, wie Polyäthylenoxyd oder die Polymerisate, die sich von Polyglycidylverbindungen ableiten.

6. Polyacetale, wie Polyoxymethylen und Polyoxyäthylen, sowie solche Polyoxymethylene, die als Comonomeres Aethylenoxyd enthalten.

7. Polyphenylenoxyde.

8. Polyurethane und Polyharnstoffe.

9. Polycarbonate.

10. Polysulfone.

11. Polyamide und Copolyamide, die sich von Diaminen und Dicarbonsäuren und/oder von Aminocarbonsäuren oder den entsprechenden Lactamen ableiten, wie Polyamid 6, Polyamid 6/6, Polyamid 6/10, Polyamid 11, Polyamid 12, ist eine bevorzugte Gruppe von Kunststoffen.

12. Vernetzte Polymerisate, die sich von Aldehyden einerseits und Phenolen, Harnstoffen und Melaminen andererseits ableiten, wie Phenol-Formaldehyd-, Harnstoff-Formaldehyd- und Melamin-Formaldehydharze.

13. Alkydharze, wie Glycerin-Phthalsäure-Harze und deren Gemische mit Melamin-Formaldehydharzen.

14. Ungesättigte Polyesterharze, die sich von Copolyestern gesättigter und ungesättigter Dicarbonsäuren mit mehrwertigen Alkoholen, sowie Vinylverbindungen als Vernetzungsmitteln ableiten.

15. Natürliche Polymere, wie Cellulose, Gummi, sowie deren polymerhomolog chemisch abgewandelte Derivate, wie Celluloseacetate, -propionate und -butyrate, bzw. die Celluloseäther, wie Methylcellulose.

16. Thermoplastische Polyester auf der Basis aliphatischer, cycloaliphatischer und/oder aromatischer Dicarbonsäuren und aliphatischer, cycloaliphatischer und/oder aromatischer Diole.

Als technisch wichtige Gruppe von Polyestern werden besonders jene angesehen, die mindestens 25 Mol-%, vorzugsweise 40 Mol-%, Terephthalsäurereste, und mindestens 25 Mol-%, vorzugsweise 40 Mol-% Alkylendiolreste enthalten, bezogen auf den Polyester. Die linearen oder verzweigten Alkylendiolreste können 2 bis 12, vorzugsweise 2 bis 6 C-Atome enthalten und sind besonders Aethylen- oder 1,4-Butylenglykolreste. Weitere für die Polyesterherstellung bekannte Monomere sowie Polyester sind z.B. in der deutschen Offenlegungsschrift 28 51 969 beschrieben.

Bevorzugte Polymere sind die Epoxidharze, thermoplastische Polyester und Polyamide.

Die Einarbeitung der Zusätze kann beispielsweise durch Einmischen der Einzelzusätze gemeinsam oder einzeln und gegebenenfalls weitere Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels erfolgen. Die Zusätze können auch vor oder während der Polymerisation zugegeben werden. Höhere Mengen werden vorteilhaft durch Umgranulieren in einem Extruder eingearbeitet.

Als Beispiele weiterer Additive und inerter Zusätze, mit denen zusammen das Flammschutzmittel eingesetzt werden kann, sind Antioxidantien, UV-Stabilisatoren oder andere Lichtschutzmittel, Weichmacher, Fliessmittel, Entformungshilfsmittel, Kristallkeimbildungsmittel, optische Aufheller, Mattierungsmittel, Farbstoffe und Pigmente, inerte oder verstärkende Füllstoffe wie Russ, Talk, Kaolin, Metallpulver, Wollastonit, Glaskugeln oder -pulver, Quarzmehl, Asbest- und Glasfasern, Dispergierhilfsmittel.

Aus den erfindungsgemässen Formmassen können Formteile aller Art hergestellt werden. Die nachfolgenden Beispiele erläutern die Erfindung näher.

A) Herstellung der Ausgangsprodukte

Herstellung von 1,3-Diglycidyl-4,5,6,7-tetrabrombenzimidazolon 44,97 g Tetrabrombenzimidazolon, 370,12 g Epichlorhydrin und 0,66 g Tetramethylammoniumchlorid werden in einem Glaskolben mit Rührer, Rückflusskühler und Stickstoffeinleitung unter Rühren auf eine Temperatur von 140°C erhitzt, wobei bei einer Innentemperatur von 104°C sich ein schwacher Rückfluss einstellt. Ueber einen Wasserabscheider

wird entstehendes Wasser abgeschieden. Nach 2 1/2 Stunden wird ein Vakuum von 55 mbar angelegt und innerhalb von 30 Minuten 150 ml eines Azeotrops von Wasser und Epichlorhydrin abdestilliert. Diese Menge wird durch reines Epichlorhydrin wieder ersetzt. Die Innentemperatur während dieses Vorganges betrug 55°C.

Nach Zusatz von 8,8 g NaOH in wässriger Lösung innerhalb von 2 Stunden wird noch 15 Minuten nachreagieren gelassen und dann auf Zimmertemperatur abgekühlt. Nach Absaugen des ausgefallenen Niederschlages und weiter in üblicher Weise vorgenommener Aufarbeitung werden 37 g (67,3 % d.Th.) von 1,3-Diglycidyl-4,5,6,7-tetrabrombenzimidazolon mit einem Epoxygehalt von 89 % und einem Schmelzpunkt von 195 - 200°C gewonnen. Nach zweimaligem Umkristallisieren wird ein Produkt mit 100 % Epoxygehalt und einem Schmelzpunkt von 206°C erhalten.

B) <u>Herstellung der oligomeren Epoxidharze</u>

<u>Beispiel 1</u>: 5,49 g der gemäss A) hergestellten Diglycidylverbindung, 3,91 g Tetrabrombenzimidazolon und 25 ml Dimethylformamid sowie ein Tropfen einer 20 %igen Lösung von 2-Phenylimidazol in n-Butanol werden in einem Glaskolben mit Rührer, Stickstoffeinleitung und Rückflusskühler innerhalb von 2 Stunden auf 170°C erhitzt. Es wird dann eine weitere Stunde bei 180°C gerührt und dann langsam im Vakuum das Lösungsmittel abdestilliert. Abschliessend wird noch eine weitere halbe Stunde bei 200°C ausreagieren gelassen. Das so entstandene Produkt wird über Nacht bei 180°C und 1 mbar getrocknet. Das so erhaltene Produkt hat eine relative Viskosität von 1,04 und einen thermogravimetrisch gemessenen Zersetzungspunkt von 295°C. Das Maximum der Zersetzung lag bei 360°C.

<u>Beispiel 2</u>: 12,62 g der gemäss A) hergestellten Diglycidylverbindung und 10,92 g Tetrabrombisphenol A sowie ein kleiner Tropfen einer 20 %igen 2-Phenylimidazollösung werden innerhalb von 1 1/2 Stunden

unter Rühren auf 230°C erwärmt und die gebildete Schmelze noch weitere 10 Minuten bei dieser Temperatur unter Vakuum (mbar) gerührt. Nach Belüften mit Stickstoff wird ein Produkt mit einer relativen Viskosität von 1,10 und einer Einfriertemperatur von 166°C erhalten. Die thermogravimetrische Analyse ergab einen Zersetzungsbeginn von 300°C und ein Zersetzungsmaximum von 340°C.

C) Anwendungsbeispiel

Mit Hilfe eines Extruders wird eine Schmelzmischung aus folgenden Komponenten hergestellt und diese dann zu Prüfkörpern verspritzt:

53 Gew.-% Poly-1,4-butylenterephthalat mit einer Intrinsic-Viskosität von 0,90 dl/g, 30 Gew.-% 6 mm lange Glasfasern, 5 Gew.-% Antimontrioxid und 12 Gew.-% des nach Beispiel 1 hergestellten Produktes.

An den Prüfkörpern (Normkleinstab) werden die in nachfolgender Tabelle aufgeführten Eigenschaften gemessen.

Tabelle

| | | | |
|---|---|---|---|
| Zugfestigkeit<br>DIN 53 455 | $N/nm^2$ | | 115 |
| Bruchdehnung<br>DIN 53 455 | % | | 1,8 |
| Biegefestigkeit<br>DIN 53 452 | $N/mm^2$ | | 180 |
| Zug-E-Modul<br>DIN 53 457 | $N/mm^2$ | | 10000 |
| Biege-E-Modul<br>ASTM D 790 | $N/mm^2$ | | 8800 |
| Schlagzähigkeit<br>DIN 53 453 | $kJ/m^2$ | 23°C | 28,5 |
| Kerbschlagzähigkeit<br>DIN 53 453 | $kJ/m^2$ | 23°C | 6,8 |
| Migrationsbeständigkeit<br>nach 10 Tagen bei 160°C | | | keine<br>Auskreidung |
| Brennbarkeit 0,16 cm Stab<br>UL 94 | | | V-0 |
| Intr.-Visc. dl/g | | | 0,79 |

Die Zähigkeitseigenschaften sind für eine flammwidrig ausgerüstete Formmasse als besonders gut zu bezeichnen.

Patentansprüche

1. Halogenhaltige, im wesentlichen lineare oligomere Epoxidharze der Formel I

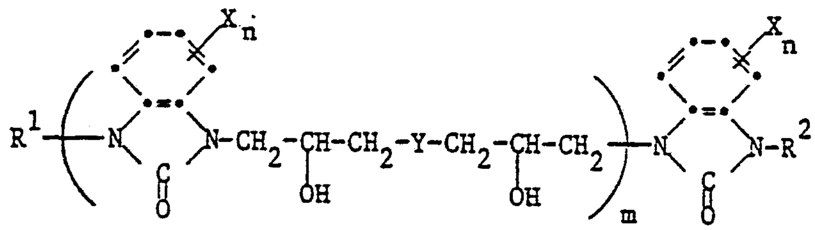

(I)

worin n die Zahlen 2, 3 oder 4 und m ein Zahlenwert von 1 bis 30 sind, X für Chlor oder Brom steht, Y den zweiwertigen Rest einer H-aciden Verbindung aus der Gruppe der Diole, zweiwertigen Phenole, Bisphenole, Dicarbonsäuren oder cyclischen Ureide darstellt und $R^1$ und $R^2$ für ein H-Atom, $-CH_2-\underbrace{CH-CH_2}_{O}$ oder $-CH_2-\underset{OH}{CH}-CH_2-Y-H$ stehen.

2. Epoxidharze gemäss Anspruch 1, dadurch gekennzeichnet, dass n die Zahlen 3 oder besonders 4 bedeutet.

3. Epoxidharze gemäss Anspruch 1, dadurch gekennzeichnet, dass m ein Zahlenwert von 1 bis 20, besonders 1 bis 10 ist.

4. Epoxidharze gemäss Anspruch 1, dadurch gekennzeichnet, dass X für Brom steht.

5. Epoxidharze gemäss Anspruch 1, dadurch gekennzeichnet, dass Y als zweiwertiger Rest eines Dioles 2 bis 12 C-Atome und als zweiwertiger Rest einer Dicarbonsäure 2 bis 20 C-Atome enthält.

6. Epoxidharze gemäss Anspruch 1, dadurch gekennzeichnet, dass Y als zweiwertiger Rest eines Bisphenoles der Formel II entspricht,

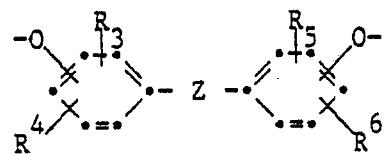 (II)

worin $R^3$, $R^4$, $R^5$ und $R^6$ unabhängig voneinander ein Wasserstoffatom, Alkyl mit 1-4 C-Atomen, ein Chlor- oder ein Bromatom darstellt und Z für eine direkte Bindung, O, S, SO, $SO_2$, $CO_2$, Alkylen mit 1 bis 8 C-Atomen, Alkyliden mit 2-8 C-Atomen oder Cylcoalkyliden mit 5 oder 6 Ring-C-Atomen steht.

7. Epoxidharze gemäss Anspruch 1, dadurch gekennzeichnet, dass Y als zweiwertiger Rest eines cyclischen Ureides den Formeln III-VII entspricht,

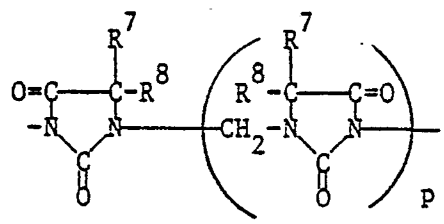 (III), 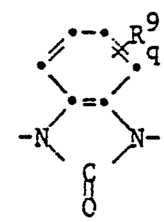 (IV)

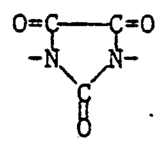 (V), 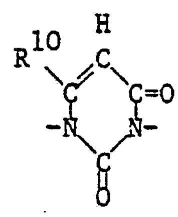 (VI), 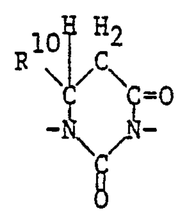 (VII),

worin p 0 oder 1 und q 0 oder ganze Zahlen von 1-4 bedeuten, $R^7$ oder $R^8$ ein Wasserstoffatom, Phenyl oder $C_1$-$C_{12}$-Alkyl oder $R^7$ und $R^8$ zusammen Tetra- oder Pentamethylen sind, $R^9$ Methyl, Chlor oder Brom bedeutet und $R^{10}$ für ein Wasserstoffatom oder $C_1$-$C_4$ Alkyl steht.

8. Epoxidharze gemäss Anspruch 1, dadurch gekennzeichnet, dass in Formel I n = 4 und m ein Zahlenwert von 1-10 sind, X für Brom steht und Y einen Rest der Formel IV darstellt, worin $R^9$ für Chlor oder besonders für Brom steht und q die Zahlen 3 und besonders 4 bedeutet.

9. Flammwidrige Kunststoffmasse, enthaltend einen duroplastischen

oder thermoplastischen Kunststoff und eine wirksame Menge eines Epoxidharzes der Formel I gemäss Anspruch 1 als Flammschutzmittel.

10. Verwendung von Epoxidharzen der Formel I gemäss Anspruch 1 als Flammschutzmittel für Kunststoffe.

Europäisches Patentamt

# EUROPÄISCHER RECHERCHENBERICHT

**0043346**
Nummer der Anmeldung

EP 81 81 0257.6

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 300 010 (CIBA-GEIGY)<br>* Anspruch 8; Seite 7 *<br>-- | 1 |
| | DE - A1 - 2 453 326 (CIBA-GEIGY)<br>* Seiten 6, 7 *<br>-- | 9,10 |
| P | EP - A1 - 0 025 018 (CIBA-GEIGY)<br>* Anspruch 1 *<br>---- | 9,10 |

**KLASSIFIKATION DER ANMELDUNG (Int. Cl.³)**

C 08 G 59/30
C 08 K 5/34
C 07 D 235/26

**RECHERCHIERTE SACHGEBIETE (Int. Cl.³)**

C 07 D 235/26
C 08 G 59/26
C 08 G 59/30
C 08 K 5/34

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 24-09-1981 | FROELICH |